# EUROPEAN PATENT SPECIFICATION

(11) **EP 1 132 097 B1**
(45) Date of publication and mention of the grant of the patent: **03.05.2006**
(21) Application number: 01107561.1
(22) Date of filing: 16.09.1994
(51) Int. Cl.: A61K 47/48

(54) **Cellular and serum protein anchors and conjugates**
Zell- und Serumproteinanker und Konjugate
Proteine serique et cellulaire d'ancrage et conjugues

(30) Priority: 15.10.1993 US 137821; 03.05.1994 US 237346
(43) Date of publication of application: 12.09.2001
(62) Divisional of application: 94930447.1
(73) Proprietor: ConjuChem, Inc., Montreal, Québec (CA)
(72) Inventor: Pouletty, Philippe, Atherton, CA 94027 (US); Pouletty, Christine, Atherton, CA 94027 (US)
(74) Representative: Walton, Seán Malcolm

(56) References cited:
- EP-A- 0 395 918
- EP-A- 0 510 949
- EP-A- 0 602 290
- WO-A1-92/05748
- DE-A- 4 014 540
- GB-A- 2 218 100
- US-A- 5 177 059
- SAMOKHIN G P ET AL: "Red blood cell targeting to collagen-coated surfaces" FEBS LETTERS, ELSEVIER SCIENCE PUBLISHERS, AMSTERDAM, NL, vol. 2, no. 154, 1983, pages 257-261, XP002076124 ISSN: 0014-5793
- MAGNANI MAURO ET AL: "Red Blood Cells as an Antigen-Delivery System" BIOTECHNOLOGY AND APPLIED BIOCHEMISTRY, ACADEMIC PRESS, US, no. 16, 1992, pages 188-192, XP002076123 ISSN: 0885-4513
- PATENT ABSTRACTS OF JAPAN vol. 130, no. 003 (C-557) 06 January 1989 & JP 63 215 642 A (NIPPON HAIPOTSUKUSU:KK) 08 September 1988
- PATENT ABSTRACTS OF JAPAN vol. 006, no. 202 (C-129) 13 October 1982 & JP 57 109 724 A (CHUGAI SEIYAKU KK) 08 July 1982
- PATENT ABSTRACTS OF JAPAN vol. 005, no. 106 (C-062) 10 July 1981 & JP 56 046 820 A (CHUGAI PHARMACEUT CO LTD) 28 April 1981

## Description

### INTRODUCTION

### Technical Field

The field of this invention is the extended lifetime of physiologically active agents in a host.

### Background

It is frequently desirable to limit the biologic effects of agents present in the mammalian blood stream. For example in the case of exposure to a toxin/poison, acute mitigation of the effects of the toxin/poison may be indicated. Alternatively, the agent may be cellular as an infected, auto-, allo-, or xenoreactive or tumorous host cell or an infectious organism.

Present blood therapies involve removing the agent from the blood by for example, plasmapheresis or adsorption with activated charcoal, disruption of the agent with for instance, antibiotics or enzymes, or the selective binding of the target, for example with antibodies. These therapies are normally employed after the patient has been exposed to the deleterious effects of the toxin/poison or the cells, so that substantial damage to the host has already occurred. In many situations, there is a need for long time administration of a drug, so as to maintain a therapeutic level in the blood stream. The oral administration or intravenous injection of drugs can result in subtherapeutic dosages for extended periods of time, followed by dosages exceeding the therapeutic level and involving serious side effects. Also, there are situations where one wishes to maintain an agent in the blood stream for an extended period of time, for example, where one wishes to obtain a strong immune response, where the immunogen is continuously present in a form which provides for continual stimulation of the immune system.

There is, therefore, substantial interest in being able to provide for improved methods of providing for the continued maintenance of agents in the blood stream, where the function of the agent may be for limiting the toxicity or pathogenicity of blood-borne agents, long term maintenance of a physiologically active agent, or long term delivery of an active agent.

### SUMMARY OF THE INVENTION

Bifunctional reagents or conjugates are provided comprising an anchor and a target binding member where the reagent is bound through the anchor to a long-lived moiety associated with the blood, either cellular or a mobile blood protein, so to provide for long-lived maintenance of the target binding member in the host. These reagents find broad applications in prophylactic and therapeutic applications, production of antibodies, reduction of the biologically effective concentration or activity of an endogenous or exogenous blood component, and in other situations where long term administration of a physiologically active compound is of interest.

Targets may be host derived or foreign. Host targets include cells and blood compounds present in undesirable concentrations, such as auto-, allo- or xenoreactive white cells, i.e. leukocytes, including macrophages, infected cells, platelets, tumorous cells, and overexpressed cytokines and hormones. Foreign targets include toxins, poisons, drugs of abuse, pathogenic infectious microbes, or the like.

The long-lived blood associated components include long-lived serum proteins, erythrocytes, platelets or endothelial cells, where the anchor binds non-specifically, to one or more surface membrane proteins, predominantly associated with the particular cell, or to a soluble protein. In the case of erythrocytes, platelets and proteins, the conjugate is administered *in vivo*. In the case of endothelial cells, the conjugate is also be administered *in vivo.*

### DESCRIPTION OF THE SPECIFIC EMBODIMENTS

Methods and compositions are provided for extending the life of a target binding member, which is a physiologically active agent, in a mammalian host by providing for binding said target binding member to a long-lived blood associated component, normally a protein. A reagent or conjugate is used which will have at least two active moieties: (1) an anchor for binding the reagent to the long-lived blood associated component; and (2) a target binding member, which is bound, directly or indirectly, to the long-lived blood associated component by means of the anchor. The long-lived blood associated component may be cellular, e.g. a mobile cell, such as an erythrocyte or platelet, namely, anuclear cells, or a fixed cell, such as an endothelial cell; or soluble or mobile proteins which are present in the blood for extended periods of time and are present in a minimum concentration of at least about 0.1 µg/ml. Proteins which fulfill this concentration requirement include, serum albumin, transferrin, ferritin and immunoglobulins, particularly IgM and IgG. The half-life of the protein should be at least about 12 hours. The target binding member may be a physiologically active agent and may provide for a wide variety of functions. It may serve as an immunogen for the production of antisera or monoclonal antibodies, where one is interested in a diagnostic or therapeutic reagent, for the production of vaccines, or for the protection, prophylactic or therapeutic against a deleterious blood-borne target, such as a pathogen, noxious agent or endogenous factor. The target binding member may react directly with the deleterious blood-borne agent, have antiproliferative activity, particularly cytotoxic activity, by itself or in conjunction with an endogenous system, such as with antibodies to a pathogen or deleterious endogenous cell, e.g. T cell, or to a toxin, may have specific binding activity with a surface membrane receptor, so as to reduce or inhibit the signal transduced by such receptor, may bind to a homing receptor, integrin or addressin, so as to inhibit extravasation or diapedesis, or may change the distribution of the target, including enhancing the excretion of the target by the host or reducing the concentration of the target in one compartment of the body in contrast to a different compartment of the body.

The target binding member is provided at a biologically effective concentration, where the target binding member may act in the bound form as part of the reagent or may act independently of the long-lived blood associated entity, as a soluble agent after release from the long lived blood component. The long-lived blood component may be part of the physiological activity, for example, where erythrocyte binding to the target may enhance the excretory processing of the target.

"Biologically effective concentration" of the physiologically active agent means the agent concentration that is bioavailable to the target during the course of the procedure. Biologically effective concentration of the target will mean the immediately bioavailable concentration of the target at the *in vivo* site of its action. For the most part, the target will be at least partially dispersed in the blood stream, may be in solution or associated with components of the blood stream. Thus the effective concentration of the target may be reduced by limiting the volume of distribution of the target as to sites at which the target is segregated, by limiting diffusion, mobility, or migration of the target, etc. Thus the target binding member will interact with a component in the blood stream, so as to provide the desired result, where the interaction may be direct or indirect to achieve the desired result, or may be a specific or non-specific interaction, where for the most part a non-specific interaction will provide for a specific result with a subset of the entities with which the target binding member interact.

The disclosed therapeutic methods are applicable to a broad range of targets, both host derived and foreign (meaning exogenous or non-host), which may be present in the blood and have a deleterious physiological effect, due to an undesirably high effective concentration, or as in the case of neoplastic cells, being present in any amount. Host derived cellular targets include, (with parenthetical clinical indication): T cell or subsets, such as αIFN+, CD4+, CD8+, LFA1+ cells (autoimmune disease, alloreactivity, xenoreactivity and inflammation), B cells or subsets such as pre-B cells, CD5+, IgE+, IgM+. (B cell lymphoma, xenograft, autoimmunity, anaphylaxy), leukocytes, such as macrophages and monocytes (inflammation, myelomonocytic leukemia), other leukocytes such as neutrophils, basophils, NK cells, eosinophils, or allo- or xeno-reactive leukocytes. (inflammation, anaphylaxis, transplant rejection), stem cells such as CD34+ cells (polycythemia), fetal red cells, such as Rh+ red cells (prophylaxis of anti-Rh immunization after pregnancy in a Rh- mother), malignant cells (malignancies; CALLA) or infected cells, particularly retroviral, e.g. HIV, infected host cells).

Host derived non-cellular targets include soluble HLA, class I and class II, and non-classical class I HLA (E, F and G) for modulating immunoregulation, soluble T or B cell surface proteins, cytokines, interleukins and growth factors such as IL1, 2, 3, 4, 6, 10, 13, 14 and 15, soluble IL2 receptor, M-CSF, G-CSF, GM-CSF, platelet growth factors, alpha, beta, and gamma- interferons, TNF, NGFs, arachadonic acid metabolites such as prostaglandins, leukotrienes, thromboxane and prostacyclin for cardiovascular diseases, immunoglobulins such as total IgE for anaphylaxy, specific anti-allergen IgE, auto or allo-antibodies for autoimmunity or allo- or xenoimmunity, Ig Fc receptors or Fc receptor binding factors, carbohydrates (gal), natural antibodies involved in allo- or xenorejection, erythropoietin, angiogenesis factors, adhesion molecules, MIF, MAF, complement factors (classical and alternate pathways, including regulatory factors), PAF, ions such as calcium, potassium, magnesium, aluminum, iron, etc, enzymes such as proteases, kinases, phosphatases, DNAses, RNAses, lipases and other enzymes affecting cholesterol and other lipid metabolism, esterases, dehydrogenases, oxidases, hydrolases, sulphatases, cyclases, transferases, transaminases, atriopeptidases, carboxylases and decarboxylases and their natural substrates or analogs, superoxide dismutase, hormones such as TSH, FSH, LH, thyroxine (T4 and T3), renin, insulin, apolipoproteins, LDL, VLDL, cortisol, aldosterone, estriol, estradiol, progesterone, testosterone, dehydroepiandrosterone (DHEA) and its sulfate (DHEA-S), calcitonin, parathyroid hormone (PTH), human growth hormone (hGH), vasopressin and antidiuretic hormone (ADH), prolactin, ACTH, LHRH, THRH, VIP, cathecolamines (adrenaline, vanillylmandelic acid, etc.), bradykinins and corresponding prohormones, metabolites, ligands or natural cell or soluble receptors thereof, cofactors including atrionatriuretic factor (ANF), vitamins A, B, C, D, E and K, serotonin, coagulation factors, e.g. prothrombin, thrombin, fibrin, fibrinogen, Factor VIII, Factor XI, Willebrand factor, plasminogen factors, e.g. plasmin, complement activation factors, LDL and ligands thereof, uric acid. In some instances one may provide specific effects associated with complement, by having inhibitors such as DAF, CD59., compounds regulating coagulation, such as hirudin, hirulog, hementin, TPA. or other compounds, such as tissue factor, nucleic acids for gene therapy., compounds which are enzyme antagonists, compounds binding ligands, such as cytokines, hormones, inflammation factors (PAF, complementation factors).

Foreign targets include drugs, especially drugs subject to abuse such as heroin and other opiates, PCP, barbiturates, cocaine and derivatives thereof, benzodiazepins, ecstasy., poisons, toxins such as heavy metals like mercury and lead, chemotherapeutic agents, paracetamol, digoxin, free radicals, arsenic, bacterial toxins such as LPS and other gram negative toxins, Staphylococcus toxins, Toxin A, Tetanus toxins, Diphtheria toxin and Pertussis toxins, plant and marine toxins, snake and other venoms, virulence factors, such as aerobactins, radioactive compounds or pathogenic microbes or fragments thereof, including infectious viruses, such as hepatitis , A, B, C, E and delta, CMV, HSV (types 1, 2 & 6), EBV, varisella zoster virus (VZV), HIV-1, -2 and other retroviruses, adenovirus, rotavirus, influenzae, rhinovirus, parvovirus, rubella, measles, polio, reovirus, orthomixovirus, paramyxovirus, papovavirus, poxvirus and picornavirus, prions, plasmodia tissue factor, protists such as toxoplasma, filaria, kala-azar, bilharziose, entamoeba histolitica and giardia, and bacteria, particularly gram-negative bacteria responsible for sepsis and nosocomial infections such as E. coli, Acynetobacter, Pseudomonas, Proteus and Klebsiella, but also gram positive bacteria such as staphylococcus, streptococcus, etc. Meningococcus and Mycobacteria, Chlamydiae, Legionnella and Anaerobes, fungi such as Candida, Pneumocystis carini, and Aspergillus, and Mycoplasma such as Hominis and Ureaplasma urealyticum.

As already indicated, the subject invention may be used for the production of antibodies, where the antibodies are harvested or splenocytes or other B cells are used for immortalization and the production of monoclonal antibodies. Thus, the antibodies may be directed to many of the targets indicated above or may be directed to various drugs, for therapeutic dosage monitoring, treatments for overdosage of drugs or drugs of abuse. Also, the subject invention may be used to vaccinate against a pathogen or other deleterious entity, where various unicellular microorganisms and viruses have been described above. In addition, the subject invention can be employed to activate T cells toward particular targets, by providing for appropriate targets for antigen presenting cells, which will then present to the T cells or providing for direct activation of T cells

The choice of the long-lived blood component will be affected by the purpose for binding to the target. Thus depending on the choice of the long-lived blood component, the target may be rapidly segregated and excreted from the body, segregated in a biologically inactive form to be slowly eliminated, or degraded over time. The long-lived blood component may be fixed or mobile, that is, substantially fixed in position, as in the case of the endothelial cells, or mobile in the vascular system, having a substantially uniform or variant distribution in the vascular system, where the long-lived blood component may be preferentially present in particular compartments, including solid tissue. The choice of long-lived blood component will also depend in part on the nature of the target. For instance, red blood cells are preferred for cellular pathogenic agents for efficient clearance, while serum protein components, such as albumin or immunoglobulins, are particularly useful for segregating soluble toxins and endothelial cells are particularly useful for restenosis and thrombosis.

A long-lived blood component has a half-life of at least about 12 hrs, usually at least about 48 hrs, preferably at least about 5 days, desirably at least about 10 days. Generally, half-lives are determined by serial measurements of whole blood, plasma or serum levels of the compound following labelling of the compound with an isotope (e.g. ¹³¹I, ¹²⁵I, Tc, 5¹ Cr ³H etc) or fluorochrome and injection of a known quantity of labelled compound I.V. Included are red blood cells (half life ca. 60 days), platelets (half life ca. 4-7 days), endothelial cells lining the blood vasculature, and long lived blood serum proteins, such as albumin, steroid binding proteins, ferritin, α-2-macroglobulin, transferrin, thyroxin binding protein, immunoglobulins, especially IgG. In addition to preferred half-lives, the subject components are preferably in cell count or concentration sufficient to allow binding of therapeutically useful amounts of the conjugate. For cellular long lived blood components, cell counts of at least 2,000/µl and serum protein concentrations of at least 1µg/ml, usually at least about 0.01 mg/ml, more usually at least about 1 mg/ml, are preferred.

The selected cellular long-lived blood components are present in high number in the vascular system. Platelets are present in from about 1-4x10⁵/µl, while red blood cells are present in about 4-6x10⁶/µl. The cells have a long half-life. The erythrocyte and platelets lack a nucleus and cell division capability. Preferred cells have a wide distribution in capillaries and tissue and express specific binding sites on the cell surface associated with their specific differentiation.

The choice of the long-lived blood component also depends on the desired route of limiting the target's toxicity/pathogenicity. For instance, it is often desirable to modulate a target's access to the central nervous system, interstitial spacers to limit toxicity, infection. By selecting the long-lived blood component based on location, distribution, specific surface markers, or other differentiating characteristic associated with the long-lived blood component, one can modulate the ultimate sites and volume of distribution of the target. For example, in some situations cellular activation results in up-regulation of a surface membrane protein. For endothelial cells, addressins, e.g. ELAM-1, or integrins, e.g. VLA-4, may be up-regulated in the case of inflammation. In this way, the agent may be segregated to a site of inflammation or lymphoid organ such as the spleen to enhance the agents exposure to a concentrated immune response including phagocytic white cells. In another example, the immune complexes of a complement binding antibody can provide for complement mediated of lysis cells or microorganism. Another example is the modification of the distribution of an endogenous or exogenous agent between compartments, such as the CNS and the blood. By providing for high affinity receptors for the agent in the blood, the concentration of the agent in the CNS may be reducecd in proportion to the binding capacity of the blood for the agent. In this way, one may be able to reduce the concentration of a drug in the brain, by enhancing the binding capacity of the blood for the drug, e.g. using antibodies for the drug, so as to withdraw the drug across the blood-brain barrier into the blood. The conjugate may be bound to the long-lived blood component non-specifically, involving covalent bonding.

The conjugate may take many forms, being comprised of one member. In the single member conjugate, both the anchor and the target binding moiety will be bound together when administered. The two moieties will be connected by covalent bonds, The anchor may be non-specific. As indicated, one may use reactive compounds, which will non-specifically react with active functionalities on the long-lived blood component as well as other molecules present in the host blood stream. In this situation, one selects a long-lived blood component which is in very high concentration in the blood stream, as compared to most other components, is present under conditions where it is reactive with the anchor, and because of its long life, within a short time, usually fewer than 7, more usually fewer than 5 days, the first binding member will be primarily associated with the desired long-lived blood component in the host.
In some instances, it will be desirable to allow for the consecutive addition of first and second compounds, where the first compound has the anchor and the second compound has the target binding member. The first and second compounds also have the complementary binding members of a specific binding pair, so that upon addition of the second compound to the first compound, the two compounds will be non-covalently bound to provide the conjugate. The first compound will comprise an active functionality, a linking group, and the first binding entity. The functionalities which are available on proteins are primarily amino groups, carboxyl groups and thiol groups. While any of these may be used as the target of the reactive functionality, for the most part, bonds to amino groups will be employed, particularly formation of amide bonds. To form amide bonds, one may use a wide variety of active carboxyl groups, particularly esters, where the hydroxyl moiety is physiologically acceptable at the levels required. While a number of different hydroxyl groups may be employed, the most convenient will be N-hydroxysuccinimide, and N-hydroxy sulfosuccinimide, although other alcohols, which are functional in an aqueous medium such as blood, may also be employed. In some cases, special reagents find use, such as azido, diazo, carbodiimide anhydride, hydrazine, dialdehydes, thiol groups, or amines to form amides, esters, imines, thioethers, disulfides, substituted amines Usually, the covalent bond which is formed should be able to be maintained during the lifetime of the target binding member, unless it is intended to be the agent release site.

A large number of bifunctional compounds are available for linking to entities. Illustrative entities include: azidobenzoyl hydrazide, N-[4-(p-azidosalicylamino)butyl]-3'-[2'-pyridyldithio]propionamid), bis-sulfosuccinimidyl suberate, dimethyladipimidate, disuccinimidyltartrate, N-γ-maleimidobutyryloxysuccinimide ester, N-hydroxy sulfosuccinimidyl-4-azidobenzoate, N-succinimidyl [4-azidophenyl]-1,3'-dithiopropionate, N-succinimidyl [4-iodoacetyl]aminobenzoate, glutaraldehyde, and succinimidyl 4-[N-maleimidomethyl]cyclohexane-l-carboxylate.

When one or both of the linking groups is permanent, the linking group(s) will not be critical to this invention and any linking group which is convenient, physiologically acceptable at utilized doses, and fills the requirements of the molecule, such as being stable in the blood stream, effectively presenting the target binding member or first binding entity, allowing for ease of chemical manipulation may be employed. The linking group may be aliphatic, alicyclic, aromatic, or heterocyclic, or combinations thereof, and the selection will be primarily one of convenience. For the most part, any heteroatoms will include nitrogen, oxygen, sulfur or phosphorus. Groups which may be employed include alkylenes, arylenes, aralkylenes, cycloalkylenes.

Generally the linking group will be of from 0-30, usually 0-10, more usually of from about 0-6 atoms in the chain, where the chain will include carbon and any of the heteroatoms indicated above. For the most part, the linking group will be straight chain or cyclic, since there will normally be no benefit from side groups. The length of the linking group will vary, particularly with the nature of the target binding member and the first binding entity, since in some instances, the target binding member or the first binding entity may naturally have a chain or functionality associated with it. In some instances, amino acids, generally from 1-3 amino acids may serve as the linking chain, particularly where the carboxyl group of the amino acid may be the reactive functionality. Thus, the amino group may serve to bond to the target binding member or first binding entity.

The length of the arms may be used to provide for flexibility, rigidity, polyfunctionality, orientation, or other characteristic for improved function of the molecule. The covalent linking group may be a functionality which has an unequal affinity for different blood proteins, or which has a high affinity for a given protein epitope or sequence, such as an IgG or albumin epitope.

The first binding entity will generally be a small molecule, where the molecule is likely to minimize any immune response. Any physiologically acceptable molecule may be employed, where there is a convenient reciprocal binding member. Thus, of particular interest is biotin, where avidin may be the reciprocal binding member, but other molecules such as metal chelates, molecules mimicking a natural epitope or receptor or antibody binding site, also may find use, where the reciprocal binding member may be an antibody or a fragment thereof, particularly a Fab fragment, an enzyme, a naturally occurring receptor. Thus, the first binding entity may be a ligand for a naturally occurring receptor, a substrate for an enzyme, or a hapten with a reciprocal receptor.

The manner of producing the first compound will vary widely, depending upon the nature of the various elements comprising the first compound. The synthetic procedures will be selected so as to be simple, provide for high yields, and allow for a highly purified product. Normally, the reactive functionality will be created as the last stage, for example, with a carboxyl group, esterification to form an active ester will be the last step of the synthesis, unless one wishes to deprotect some functionality of the target binding member or the first binding entity as the last step.

Usually, the first compound when it comprises the first binding entity will have a molecular weight of at least about 200 D and not more than about 2.5 kD, usually not more than about 1.5 kD and frequently less than about 1 kD.

Illustrative compounds include N-hydroxysuccinimidyl biotin ester, N-hydroxysulfosuccinimidyl biotin ester, N-hydroxysulfosuccinimidyl ester of N-biotinyl 6-aminohexanoic acid, N-hydroxysulfosuccinimidyl ester of N-biotinyl 4-butyryl, 3-aminopropyl disulfide, and the like. A large number of water soluble biotin derivatives for functionalizing proteins are available and to the extent that such compounds have linkers which are physiologically acceptable, these compounds may find application in this invention.

The first compound will usually be administered as a bolus, but may be introduced slowly over time by infusion using metered flow. The first compound will be administered in a physiologically acceptable medium, e.g. deionized water, phosphate buffered saline, saline, mannitol, aqueous glucose, alcohol, vegetable oil. Usually a single injection will be employed although more than one injection may be used, if desired. The first compound may be administered by any convenient means, including syringe, trocar, catheter. The particular manner of administration, will vary depending upon the amount to be administered, whether a single bolus or continuous administration. For the most part the administration will be intravascularly, where the site of introduction is not critical to this invention, preferably at a site where there is rapid blood flow, e.g. intravenously, peripheral or central vein. Other routes may find use where the administration is coupled with slow release techniques or a protective matrix. The intent is that the first compound be effectively distributed in the blood, so as to be able to react with the blood components.

For the most part, reaction will be with mobile components in the blood, particularly blood proteins and cells, more particularly blood proteins and red cells. By "mobile" is intended that the component does not have a fixed situs for any extended period of time, generally not exceeding 5, more usually one minute. For the most part, reaction will be with plasma proteins, such as the immunoglobulins, particularly IgM and IgG, albumin, ferritin, and to a lesser degree other proteins which are present in substantially reduced amount. There may also be reaction with platelets, endothelial cells and white blood cells. There will, therefore, initially be a relatively heterogeneous population of functionalized proteins and cells. However, for the most part, the population within a few days will vary substantially from the initial population, depending upon the half-life of the functionalized proteins in the blood stream. Therefore, usually within about three days or more, IgG will become the predominant functionalized protein in the blood stream. This means that after a few days, the target binding member will be conjugated to, or the second compound will, for the most part, become conjugated with and bound to IgG.

In many situations, one may use a single first compound comprising the first binding entity, once such compound has been thoroughly tested in hosts, particularly human hosts, since its physiology will be well established, its pharmacokinetics will be established, and its safety over an extended period of time may be also established. In some instances the first compound will be physiologically and/or therapeutically active, where it may find use independent of addition of the second compound. However, to the extent that there may be idiosyncratic individuals, or that chronic administration of the first compound may result in some immune reaction, it may be desirable to have more than one first compound to be used for administration. However, since the role of the first compound may be somewhat restricted when used in combination with a second compound, it is not necessary that one develop numerous alternatives, although there will be numerous alternatives which will be useful for the same purpose.

For the most part, the half-life of the first compound will be at least about five days, more usually at least about 10 days and preferably 20 days or more. The period for providing an effective concentration may be much longer, since one may introduce a substantial excess of the first compound so that even after two or three half-lives, there may still be a useful amount of the first compound in the blood stream.

Generally, it will be satisfactory to have the target binding member as part of the first compound.

The dosage of the first compound will depend upon whether it comprises the target binding member and will therefore be dependent on the adverse effects of the target binding member, its activity when bound to blood components, the time ' necessary to reduce the free concentration of the first compound containing the target binding member, the dosage necessary for therapeutic activity, the indication being treated, the sensitivity of the agent to blood enzymes, the route and mode of administration. As necessary, the dosage may be determined empirically, initially using a small multiple of the therapeutic dosage normally administered, and as greater experience is obtained, enhancing the dosage. Where the target binding member is used to elicit an immune response, the target binding member will be an antigen, and will not rely on the conjugation to the blood component for enhancing the immune response. In this case, relatively large dosages may be employed, where one is interested in producing antibodies as a product, where the treated host will normally be a domestic or laboratory animal. Where the target binding member is acting as a vaccine, the dosage may be smaller and may be determined empirically. For the most part, as a vaccine the dosage will generally be in the range of about 10 ng to 100 µg. Normally, any additional dose will be administered after the original dose has been diminished by at least 50%, usually at least 90%, of the original dose.

One or more agents of interest may be present in the first compound, usually multiple agents of interest being bound through first or second linking groups to a central core, e.g. a protein, nucleic acid, polysaccharide, or other multifunctional entity. Not only does a linking group serve to covalently bond the target binding member, but it also serves to determine whether the target binding member remains bound to the blood component or if released, the manner and rate of release. Therefore, the nature of the linking group will vary widely depending upon its role.

Where the target binding member is to be retained bound to the blood component, any of a wide variety of convenient linking groups, including a bond, may be employed. Thus, the same types of linking groups employed in the first compound will find acceptance for the second compound. However, where the linking group is to be cleaved and release the target binding member, the linking group will vary depending upon the nature of the target binding member, the desired rate of release, the valency or the functionality on the target binding member which is to be released. Thus, various groups may be employed, where the environment of the blood, components of the blood, particularly enzymes, activity in the liver, or other agent may result in the cleavage of the linking group with release of the target binding member at a reasonable rate.

Functionalities which may find use include esters, either organic or inorganic acids, particularly carboxyl groups or phosphate groups, disulfides, peptide or nucleotide linkages, particularly peptide or nucleotide linkages which are susceptible to trypsin, thrombin, nucleases, esterases, acetals, ethers, particularly saccharidic ethers. Generally, the linking group for cleavage will require at least two atoms in the chain, e.g. disulfide, and may require 50 atoms, usually not more than about 30 atoms, preferably not more than about 20 atoms in the chain. Thus, the chain may comprise an oligopeptide, oligosaccharide, oligonucleotide, disulfide, organic divalent groups which are aliphatic, aromatic, alicyclic, heterocyclic or combinations thereof, involving esters, amides, ethers, amines The particular linking group will be selected in accordance with physiological acceptance, desired rate of cleavage, synthetic convenience.

The conjugate may be prepared in any convenient way, depending on the nature of the binding member components of the conjugate, the need to maintain the binding capability of the binding members and such other considerations as are relevant to the use of the conjugate *in vivo.* Depending on the nature of the conjugate, the coupling ratio between the two binding members of the conjugate may vary from one to having a plurality of one or both of the binding members, there usually being on the average not more than 6, more usually not more than three of either of the binding members in the conjugate. Generally the anchor will be one, but may be more than one where enhanced affinity for the long-lived blood component is desired.

As previously indicated, the anchor and the target binding member may be covalently linked by an appropriate chain, which may take many different forms.

The nature of the linkage between the binding members of the conjugate will vary widely depending upon the chemical composition of the binding members, the fate of the target binding member, the nature of the target Generally, stable linkages will be employed and covalent linkages find general applicability. By "stable" is intended that the linkage will not be preferentially cleaved as compared to other bonds in the molecule; "unstable" intends preferential cleavage. However, occasionally one may wish to permit limited time-course degradation of the linkage. For example, in some cases one may wish to have controlled release of the complex of the conjugate and the target, where the affinities of the binding members of the conjugate are very high. By providing for cleavage or degradation of the conjugate the target will be released in accordance with the rate of cleavage or degradation of the conjugate, rather than the off rate of the target from the complex. A wide variety of linkages will be unstable under physiological conditions, where the instability may be as a result of enzymatic or non-enzymatic reactions. For example, ester linkages can be provided which are relatively labile and will be cleaved over time in the blood stream. By providing for varying degrees of steric hindrance, different degrees of lability can be achieved. Alternatively, sequences can be provided, which are recognized by a wide variety of enzymes such as proteases. For example, a series of arginine-lysine dimeric units will be sensitive to trypsin. Other protease labile linkages may be employed, where the protease may be found in the bloodstream. Alternatively, one may employ disulfide linkages, which will be subject to reductive cleavage. Other functionalities which may find use include oligosaccharides, thiol esters, nucleic acids. Alternatively, one may rely upon the nature of one or both of binding members to provide for release of the target from the long-lived blood component, e.g. peptide nature of antibodies subject to proteases.

There are extensive reports in the literature of procedures for covalently joining a wide variety of compounds to prepare conjugates with different functional groups. Various synthetic schemes may be envisioned, again depending upon the nature of the conjugate binding members. Thus, where sulfhydryl groups are present, these may be readily activated to provide for linkage to a sulfhydryl group to provide a disulfide or with a maleimide group, where a thiol may bind to the maleimide to provide for a thioether. Carboxyl groups may be readily activated with a wide variety of hydroxyl compounds or carbodiimide, where the ester or anhydride is allowed to react with hydroxyl or amino groups to provide esters or amides. Analogously, saccharide moites can be activated with periodate. Other linkages may also find application, such as imines, hydrazines, etc. Alternatively, non-covalent linkages may be used, such as biotin-avidin linkages, double antibody linkages, lectinsaccharide linkages. Where the conjugate binding members are both proteins, such as antibodies or fragments, the conjugate may be genetically engineered as a single construct, cloned and expressed using a variety of vectors and host cells. Alternatively, the two conjugate binding members can be recombinantly or chemically synthesized and subsequently coupled, particularly by affording unique functionalities available for coupling. Chemical synthesis may employ commercially available synthesizers using liquid or solid phase synthesis, where the synthesis may be all or a part of the conjugate. The subject compositions may be used for the treatment, prophylactic or therapeutic, of a wide variety of cellular diseases, toxicities and environmental exposures and may be administered in a wide variety of ways, depending on the indication. If desired, the conjugate may be first bound to the long-lived blood component in appropriate proportion followed by administration. Alternatively, the conjugate may be administered to the host for binding to the long-lived blood component cells or proteins. The amount of the conjugate which is administered will vary widely, depending upon the nature of the conjugate, the purpose for the conjugate, the therapeutic dosage, the physiological activity of the compound when present as a conjugate, and when bound to a cell. Therefore, the amount of conjugate administered to a host may vary from 1 *µ*g to 50 mg/kg of host.

The subject compositions will, for the most part, be administered parenterally, such as intravascularly (IV), intraarterially, intramuscularly (IM), subcutaneously (SC). Administration will normally be by transfusion if the conjugate is bound to cells. If the conjugate is unbound, administration will normally be IV, IM or SC. Where the compositions are of low molecular weight (less than about 10 kD) or resistant to digestive enzymes, conjugate administration may be oral, nasal, rectal, transdermal or aerosol, where the nature of the conjugate allows for transfer to the vascular system. Physiologically acceptable carriers will usually be employed, such as water, saline, phosphate buffered saline, aqueous ethanol, plasma, proteinaceous solutions, glucose or mannitol solutions. The concentration of the conjugate will vary widely, generally ranging from about 1 pg/ml to 50 mg/ml. Other additives which may be included include buffers, where the media are generally buffered at a pH in the range of about 5 to 10, where the buffer will generally range in concentration from about 50 to 250 mM, salt, where the concentration of salt will generally range from about 5 to 500 mM, physiologically acceptable stabilizers. The compositions may be lyophilized for convenient storage and transport.

The choice of the long-lived blood component will affect the manner in which the biological activity of the target is modified. Depending on the nature of the target different long-lived blood components will be employed. Where the target is a molecule, such as a small organic molecule or peptide, serum proteins will be satisfactory long-lived blood components, and elimination of the target will accompany elimination of the serum protein. The target must be substantially inactivated while bound to the conjugate, particularly where the target has cytotoxic effects. Where the target is a virus particle or cell, usually the long-lived blood component will be a cell, the choice of cell depending upon whether one wishes to have the target cell rapidly eliminated or maintained in the host segregated to particular cellular compartments. For example, if one wishes to eliminate virus infected cells or neoplastic cells, the long-lived blood component would be erythrocytes or platelets, so that the target cell would become bound to the erythrocytes or platelets to prevent the target cell to enter small vessels and the complex recognized by the spleen for elimination. With erythrocytes, the deformation of the erythrocytes on binding to the target cell aids in the recognition of the complex for elimination. A similar mechanism is operative for platelets.

The subject invention may be used in chronic or acute situations, either prophylactic or therapeutic. For acute situations, the conjugate will normally be administered in anticipation of the acute situation. For example with drug addicts, one may wish to prevent the use of a particular drug of abuse, particularly where the patient is on a methadone program. By administering the subject conjugate, where the conjugate may exert an effective binding capability of weeks or months, taking of the drug of abuse would result in its inactivation, inhibiting the euphoric effect. Similarly, where one is concerned about nosocomial infection, one would administer the conjugate in anticipation of the infection, so that upon infection, the toxin or tumor necrosis factor would become bound to diminish its effective concentration below a pathological level.

For therapeutic use, one could administer the conjugate to a cancer patient, so that the blood would be monitored for metastatic cells or growth factor excess. In this case, one would use an long-lived blood component which provides for rapid elimination, such as erythrocytes. Where cells are not normally found in the vascular system, such as mammary cells, the conjugate would have as the target protein, a mammary cell surface protein, so that any mammary cells in the vascular system could be detected and eliminated. In the case of T cells involved with autoimmune disease, where such cells have a common epitope, the conjugate would supervise the blood for such cells and be able to bind to such cells and eliminate the cells by the mechanisms indicated above.

The subject invention can be used in anticipation of one or more events which may have deleterious physiological effects, e.g. drug ingestion or adverse physiological response of a host to trauma or surgery, or for a chronic situation where the continued production of toxins, virus particles as a result of an intractable infection, is continuously monitored by the conjugate bound to a long lived blood component. The subject invention provides a long-lived source of binding capability, where upon binding of a target, the deleterious biological effects of the target are rapidly diminished and the target may be slowly or rapidly eliminated from the host by a variety of natural mechanisms associated with degradation, chemical modification, elimination of modified or defective erythrocytes and platelets.

Because of the extended delivery time or availability of the subject agents, the subject invention may be used in a wide variety of situations. The target binding member may be an antibody to a toxin, where there is concern about nosocomial infection in a hospital or other situation where disease may be spread. The subject invention may be employed before surgery so as to ensure that a level of drug is maintained during and subsequent to the surgery without requiring repetitive administration, avoiding the disturbance of the patient. For example, one may use anticlotting agents, where the nature of the surgery and indication is susceptible to the formation of clots. One may use inhibitors of leukocyte homing to prevent perfusion injury. One may use the subject invention with cardiovascular drugs, where a patient is particularly susceptible during an extended period to myocardial infarction. Other treatments which will benefit from long term availability of drugs include hormonotherapy, infertility therapy, immunosuppressive therapy, neuroleptic therapy, drug of abuse prophylaxy, treatment of diseases caused by infectious agents, treatment of hemophilia.

By conjugating a biologically active target binding member to IgG in the blood of a mammalian host, many advantages ensue, in providing for a new activity for the immunoglobulins, while retaining many of the desirable features of the immunoglobulins, in addition to the extended life-time. For example, the immunoglobulins may still have F_{c} effector function, such as its role in complement fixation, or the action of antibody dependent cytotoxic cells, effect on inactivation and secretion. Similarly, serum albumin and other long lived serum proteins can act to inactivate target entities and aid in their rapid elimination. Thus, the- blood components to which the target binding member is bound impart their physiological activities to the activity of the target binding member. In this way cellular targets may be inactivated or eliminated by having immunoglobulins directed to a cellular or soluble target or by coating the cellular or soluble target with blood proteins.

The following examples are offered by way of illustration and not by way of limitation.

### EXPERIMENTAL

### Comparative Example 1: Modification of Cocaine Volume of Distribution

A monoclonal antibody raised against a cocaine-BSA conjugate (molar ratio, 10:1) using BALB/c mice and SP20 myeloma is screened by solid phase microtiter plate ELISA against Cocaine-BSA and irrelevant peptide-BSA as control antigen. Selected antibodies specificity is verified by competition assay against cocaine-BSA in which binding of antibodies is inhibited in a dose dependent manner by free cocaine. Selected antibody is IgG1, with a supernatant titer by ELISA of 1:64. Mab is produced in ascites fluid and purified by protein A affinity chromatography.

Anti-RBC Mab is an IgG1 Mab specific for human glycophorin A and reacting with blood group antigen M (obtained from Hospital Saint-Louis, Paris). The Mab cross-reacts with chimpanzee and Macaccus muliata erythrocytes.

Fab fragments (produced by papain digestion monitored by SDS-PAGE) from both anti-RBC and anti-cocaine Mabs are conjugated using the Nakane method, in which the first member of the conjugate is activated by sodium periodate, before addition of the second member of the conjugate, resulting in a carbohydrate-NH₂ residue covalent coupling. pH is monitored during coupling reaction and ratios of both members are optimized to provide a mean molar ratio close to 1:1. Conjugate is purified by gel filtration. The pics corresponding to molecular weight between 80 KD and 120 KD (determined by SDS PAGE) are selected. Reactivity of conjugate with cocaine is verified by ELISA as above, using an anti-mouse kappa chain specific peroxidase conjugate, and as control an anti-mouse Fc specific peroxidase conjugate (which does not react with cocaine bound Fab). Reactivity of conjugate with human RBC is tested by flow cytometry using anti-mouse Fab FITC conjugate and as control an anti-mouse Fc specific FITC conjugate (which does not react with cocaine bound Fab). The conjugate is shown not to agglutinate RBCs and not to induce hemolysis (between 100 ng/mL and 10 µg/mL) when incubated in human and monkey plasma with addition of fresh rabbit complement.

The biodistribution of ¹²⁵I labelled cocaine is evaluated with and without pretreatment of animals with anti-RBC, cocaine specific conjugate. Animals (n=3) receive IV by slow injection (5 minutes) 50 µg/Kg of conjugate diluted in glucose 5% with 1% of human serum albumin. One day after the injection, animals receive 10 µcurie of ¹²⁵I-labelled cocaine IV (100 µg·per animal). 1 hour after the cocaine injection, animals are sacrificed, and various blood and tissue specimens are collected, including brain specimens (frontal and occipital cortex).

Control animals (n=3) receive only ¹²⁵I labelled cocaine and no conjugate. Specific radioactivity is measured using a gamma counter, and expressed in cpm per ml of blood or cpm per gram of tissue. Animals which receive the conjugate pre-treatment have an average brain ¹²⁵I specific activity of 2.2% (+/- 0.7%) the average brain I specific activity of control animals, and a mean whole blood specific activity of 25 times (+/- 4) that of control animals (with a ratio of erythrocyte to plasma ¹²⁵I specific activity of 20:1 in the conjugate treated group, and 0.2 in the control group).

The results demonstrate that pre-treatment with an erythrocyte bound to a conjugate specific for cocaine modifies cocaine volume of distribution, decreases diffusion of cocaine into the central nervous system, and decreases the ratio of plasma to erythrocyte cocaine concentration (with a net increase in whole blood concentration).

### Comparative Example 2: Anti-T cell Subset Conjugate

A rat anti-mouse RBC specific Fab fragment is conjugated to purified recombinant HIV1 gp120 (Molar ratio 1:2). Following IV administration to SCID Hu mice (10 µ per mouse) of the RBC/gp 120 long-lived blood component, whole blood specimens are collected serially in the following 48 hours, and analyzed by fluorescent microscopy and flow cytometry. In the first 6 hours, rosettes of erythrocytes and CD3+, CD4+, CD8- T cells are observed in the animals pretreated with the Anchor, and are absent in control animals.

### Comparative Example 3: Anti-Viral Protein Anchor

A rat anti-mouse RBC specific Fab fragment is conjugated to purified recombinant CD4 (Molar ratio 1:3). 48 hours following IV administration to BALB/c mice (10 *µ*g per mouse) of the RBC/CD4 conjugate, ¹²⁵I labelled recombinant HIV1 gp120 is injected IV to conjugate pretreated or control animals. Half-life (whole blood, erythrocyte and plasma) concentration of gp120 is measured in the following 24 hours and 8 days. The plasma half-life of gp120 is dramatically decreased in treated animals, whereas the erythrocyte and whole blood half lifes are increased.

### Example 4: Two-stage Addition of Lifetime Extended Immunogen

I. Two rabbits were injected with a solution of N-succinimidyl biotin ester (NHS-biotin)(5 or 50 mg in 200 µl or 1 ml of DMSO) i.v. on day 0. Blood samples were taken at initially hourly, followed by daily intervals and the samples analyzed for the presence of biotin by gel electrophoresis (SDS-PAGE) employing 60µg of sample, detecting the proteins to which biotin is bound by avidin-peroxidase conjugate, using a luminescent substrate (ECL kit, Amersham). For biotin bound to red blood cells, the cells were isolated by centrifugation, washed, lysed by hypotonic lysis, and the proteins separated by SDS-PAGE in the same manner as the plasma proteins. For the plasma proteins, the electrophoresis was run under reducing and non-reducing conditions, where the IgM and IgG were reduced to sub-units under the reducing conditions.

The gels for the plasma proteins of one of the rabbits showed that biotin was bound to IgM, IgG, p90, p75 (transferrin), serum albumin, and p38. The duration for serum albumin for detection in the gel electrophoresis under reducing conditions was 9 to 12 days for the serum albumin, and up to 33 days for IgM and IgG, while for the non-reducing conditions, where the time for exposure was substantially less, bands could be observed for the serum albumin for 2 days and for the IgG for 9 days. With the red blood cells, a number of proteins could be observed for 12 days, the next point being 33 days, at which time no bands could be observed. The major labeled component had an electrophoretic pattern suggesting Band 3.

II. To demonstrate that the subject invention can be used to enhance the immune response to a heterologous protein, after adminstration of the biotin conjugate as described above, 250 *µ*g or 1 mg of egg white avidin was injected intravenously into each of two rabbits. Within about 2 days the titer began to rise rapidly and by 10 days a high anti-avidin titer was observed by ELISA, using avidin coated to microplates, as evidenced by an OD value of about 600. By comparison, when avidin was administered as described above to rabbits to which the biotin conjugate had not been previously administered, there was substantially no production of antibodies until an intravenous booster injection of 50 µg of avidin was made on day 6, at which time there was a rapid increase in anti-avidin titer, approximating the titer observed with the biotin conjugate modified rabbits by day 12.

### Example 5. Binding of Biotin-NHS to RBCs and Plasma Proteins.

5 mg (-1.5 mg/kg) and 50 mg (- 15 mg/kg) of NHS-biotin solubilized in DMSO were injected into rabbits 3, or A and 8, respectively. Blood samples were then taken 0.5, 1, 2 and 4 h on the same day after injection and then on 1, 2, 3, 6, 9, 13, 20, 27, 34, 41, 48 and 55 days after injection.

30 minutes after injection, all RBCs were biotinylated as shown by flow cytometry performed with phycoerythrin-conjugated avidin. The mean fluorescence was much lower for rabbit 3 than for rabbit 8 (26 and 320, respectively), showing a direct relationship between the labeling of RBCs and the dose of NHS-biotin. Half-lives of 17 or 15 days were calculated from the curves obtained with rabbit 3 or 8, respectively. A life span of 55 days was obtained with rabbit 3. After 49 days, 6 % of biotinylated RBCs survived for rabbit 8. As measured by conventional methods, the life span of rabbit RBCs is 45 to 70 days.

The plasma proteins of rabbits 3 and 8 were analysed by immunoblotting. The plasma proteins were separated under reducing conditions (10 mM DTT) in a 10% polyacrylamide gel (Coomassie blue staining). Major components were identiifed as p180, p90, p75, albumin, and the heavy and light chains of immunoglobulins. All plasma proteins revealed with Ponceau red were able to capture avidin-phycoerythrin, showing that they are biotinylated. For rabbit 3, only serum albumin could be detected on day 20. The staining was much more intense with samples from rabbit8, showing biotinylation of high molecular weight components (- 200 kDa) and the light chains of immunoglobulins. No staining was observed day 19.

The pattern of plasma proteins separated under non-reducing conditions in a 8% polyacrylamide gel (Coomassie blue staining) showed IgM, IgG, p90, p75, and serum albumin as the major components. On immunoblots, albumin (60 kDa), transferrin, p90, IgG (160 kDa) and high molecular weight components corresponding in part to IgM were detected. The half-life of high molecular weight components and IgG appeared longer than that of albumin, since the latter could not be visualized from samples taken day 12 from rabbit 8.

### Example 6. Production of Anti-avidin with Avidin Bound to Biotinylated Plasma Components

A rabbit was injected on day 0 with 50mg NHS-biotin in 1 ml DMSO followed 30 min later with an injection of peroxidase labeled avidin (1 ml/250 µg). Seven days later the rabbit was injected with ¹²⁵I-avidin (1 x 10⁷cpm: 50µg). Blood samples were taken periodically thereafter for the presence of ¹²⁵I and the presence of anti-avidin antisera. On day 53, 50 mg of NHS-biotin in 600 µl DMSO was injected followed 30 min later with 1 mg/500µl of avidin and blood samples taken periodically thereafter to measure the anti-avidin level. Anti-avidin appeared as soon as day 2, with the level maximizing around day 12 and decreasing slowly thereafter. The level of antibodies was not significant after 33 days. After boosting with NHS-biotin and avidin, high titered antibodies were obtained. Based on OD value as determined using avidin coated ELISA plates, on day 70, the titer was about 8-fold greater than on day 12 (606 vs. 4790).

In a control rabbit that did not receive NHS-biotin, no significant level of antibodies was observed within 7 days after injection of 500 µg of peroxidase labeled avidin. However, a further injection of ¹²⁵I-avidin (15 µg) did result in an immuogenic response 5 days later.

The disclosed invention provides for extending the lifetime of an agent in the blood strream of a mammalian host for a wide variety of purposes. The disclosed invention provides an improved method for limiting the pathogenic or toxic effects of agents present in the mammalian blood stream. In addition the subject invention allows for continual presentation of antigens, providing molecules which can act as surveillants, or maintaining a therapeutic modality for an extended period of time to avoid repetitive administsration of a physiologically active agent.

The method finds application where surveillance is desired for the presence of physiologically detrimental agents in the blood stream. By providing for a conjugate which binds specifically to such agents and is bound to a long-lived blood associated component which enhances the life of the conjugate in the blood, the conjugate can act to bind and inactivate the agent, and by appropriate choice of the long-lived blood associated component, the agent can be slowly or rapidly eliminated from the host, using the normal physiological mechanisms for such elimination.

All publications and patent applications mentioned in this specification are indicative of the level of skill of those skilled in the art to which this invention pertains.

## Claims

1. Use of a composition for the manufacture of a medicament for use in a method of treatment of a mammalian host having a blood borne target, having a deleterious physiological effect
said composition comprising an anchor covalently linked to a target binding member,
said anchor reacting *in vivo* with an amino group, carboxyl group or thiol group of a protein to form a stable covalent bond,
said stable covalent bond being selected from the group of amide, ester, imine, thioether, disulfide and substituted amine bonds,
the *in vivo* half-life of the target binding member being extended by the formation of said stable covalent bond.

2. Use according to claim 1 wherein the target-binding member is a physiologically active agent.

3. Use according to any one of the preceding claims wherein the target-binding member comprises a peptide.

4. Use according to any one of the preceding claims wherein the anchor reacts with a thiol group of the protein

5. Use according to any one of the preceding claims, wherein the covalent bond formed by the reaction of the anchor with the protein is a thioether.

## Patentansprüche

1. Verwendung einer Zusammensetzung zur Herstellung eines Medikaments zur Verwendung in einem Verfahren zur Behandlung eines Säugetierwirts mit einem im Blut enthaltenen Target mit schädlicher physiologischer Wirkung,
wobei die Zusammensetzung einen kovalent an ein Target-Bindungselement gebundenen Anker umfasst,
wobei der Anker in vivo mit einer Aminogruppe, Carboxylgruppe oder Thiolgruppe eines Proteins reagiert, um eine stabile kovalente Bindung zu bilden,
wobei die stabile kovalente Bindung aus der aus Amid-, Ester-, Imin-, Thioether-, Disulfid- und substituierten Aminbindungen bestehenden Gruppe ausgewählt ist,
wobei die In-vivo-Halbwertszeit des Target-Bindungselements durch die Bildung der stabilen kovalenten Bindung verlängert wird.

2. Verwendung nach Anspruch 1, worin das Target-Bindungselement ein physiologisch aktives Mittel ist.

3. Verwendung nach einem der vorangegangenen Ansprüche, worin das Target-Bindungselemement ein Peptid umfasst.

4. Verwendung nach einem der vorangegangenen Ansprüche, worin der Anker mit einer Thiolgruppe des Proteins reagiert.

5. Verwendung nach einem der vorangegangenen Ansprüche, worin die durch Reaktion des Ankers mit dem Protein gebildete kovalente Bindung ein Thioether ist.

## Revendications

1. Utilisation d'une composition pour la fabrication d'un médicament à utiliser dans une méthode de traitement d'un hôte mammifère ayant une cible contenue dans le sang ayant un effet physiologique nuisible,
ladite composition comprenant un ancrage attaché de façon covalente à un membre ayant une affinité pour la cible,
ledit ancrage réagissant *in vivo* avec un groupe amino, un groupe carboxyle ou un groupe thiol d'une protéine pour former une liaison covalente stable,
ladite liaison covalente stable étant sélectionnée dans le groupe consistant en amide, ester, imine, thioéther, disulfure et liaisons amide substituées,
la demi-vie *in vivo* du membre ayant une affinité pour la cible étant prolongée par formation de ladite liaison covalente stable.

2. Utilisation selon la revendication 1 dans laquelle le membre ayant une affinité pour la cible est un agent physiologiquement actif.

3. Utilisation selon l'une quelconque des revendications précédentes dans laquelle le membre ayant une affinité pour la cible comprend un peptide.

4. Utilisation selon l'une quelconque des revendications précédentes dans laquelle l'ancrage réagit avec un groupe thiol de la protéine.

5. Utilisation selon l'une quelconque des revendications précédentes dans laquelle la liaison covalente formée par la réaction de l'ancrage avec la protéine est un thioéther.
